# EUROPEAN PATENT SPECIFICATION

(11) **EP 3 806 794 B1**
(45) Date of publication and mention of the grant of the patent: **19.04.2023**
(21) Application number: 19744780.8
(22) Date of filing: 17.06.2019
(51) Int. Cl.: A61F 13/38

(54) **SOLUBLE ARTICLES AND MANUFACTURE AND DISPOSAL THEREOF**
LÖSLICHE GEGENSTÄNDE SOWIE DEREN HERSTELLUNG UND ENTSORGUNG
ARTICLES SOLUBLES, LEUR FABRICATION ET LEUR ÉLIMINATION

(30) Priority: 15.06.2018 GB 201809891; 15.06.2018 GB 201809894; 15.06.2018 GB 201809895; 15.06.2018 GB 201809896
(43) Date of publication of application: 21.04.2021
(60) Divisional application: 22174071.5
(73) Proprietor: McCormack Innovation Limited, Balmullo St. Andrews Fife KY16 0BD (GB)
(72) Inventor: MCCORMACK, Brian James, Fife KY2 6QS (GB)
(74) Representative: Symbiosis IP Limited
(86) International application number: PCT/GB2019/051670
(87) International publication number: WO 2019/239157

(56) References cited:
- WO-A1-01/76683
- US-A- 5 709 010
- US-A- 6 080 126

## Description

A first aspect of the present invention relates to a dissolvable cotton bud.

Cotton buds are regularly used absorbent material. The material used for the bud in these cotton buds today is synthetic fibres such as polyester or nylon and the shaft of the cotton bud is made from plastics material. Such cotton buds are widely used for sanitary, medical and cosmetic purposes. The disposal of these cotton buds creates significant environmental issues. Whilst medical waste is often incinerated, most of the cotton buds either ends up in landfill or flushed down the sewage system. This creates considerable blockage problems in any sewage system. These cotton buds also end up in ocean and create extremely persistent problems as recently highlighted in the UK Press.

An aspect of the present invention seeks to address this problem.

Accordingly the present invention is directed to a cotton bud, in which a shaft is made from rolled dissolvable paper and the buds at the ends comprise entangled dissolvable cotton wool type fibres.

This provides the advantage that the whole of the cotton bud after use can be dissolved in a short time frame in water. Therefore should the cotton bud be disposed of down a sewage system providing a reasonable flow of water occurs the cotton bud will dissolve completely. Furthermore should such a cotton bud find its way into the ocean similarly the cotton bud will dissolve.

The entangled dissolvable paper fibres with cotton wool like properties are described below. This provides the advantage that the fibres are both absorbent, have a reasonable use period and dissolve in their entirety.

Advantageously the hollow shaft contains compressed entangled such paper fibres which can be pulled out when the existing bud needs to be refreshed after used. In a preferred embodiment the compressed fibres have pre-scored breaks in order to break off the used part once the fresh part has been pulled out. This provides the advantage of providing further life to the cotton bud.

In a preferred embodiment the dissolvable paper of the shaft is carboxy methyl cellulose. Advantageously it is coated with an appropriate soluble coating in order to provide added stiffness and endurance. Alternatively the rolled paper can be a multilayer paper held together by dissolvable glue. This provides the advantage of additional durability of the shaft.

Cotton wool or cotton pads are regularly used absorbent material. While sometimes sold as balls they are longer made from natural cotton fibres. This is largely caused by the United States Federal Trade Commission, which has stringent requirements for the labelling of textile products. The material used for the cotton wool today is synthetic fibres such as polyester or nylon. Such cotton wool/pads and products containing them are widely used for sanitary, medical and cosmetic purposes. The disposal of this material creates significant environmental issues. Whilst medical waste is often incinerated, most of the other material either ends up in landfill or flushed down the sewage system. This creates a considerable blockage problems in any sewage system. Cotton wool is also extremely persistent in land fill.

There is disclosed herein a method of making an absorbent cotton wool type material comprising the steps of: a) creating a blend of water soluble fibres; b) subjecting the fibres to rotational motion in a confined chamber; and c) carding the fibre conglomerations created to disentangle and intermix the fibres.

This provides the advantage of a simple method to produce a soluble fibre product with qualities similar to cotton wool.

Advantageously the soluble fibre is comprised of sodium carboxyl methyl cellulose, wood pulp or combinations thereof. This has the advantage that these fibres are readily available and will dissolve in water in a reasonable time. In a preferred embodiment the starting material is 10 point (252 gsm) Facestock calendered water soluble paper.

The confined chamber is loosely packed with the soluble fibres to allow aeration of the fibres on mixing. Advantageously further soluble material can be added to the soluble fibres to create the desired material properties.

Advantageously rotational blending with high speed cutting blades occurs for approximately 1 minute, which is repeated a number of times to achieve the required result. These times can be varied to achieve the product characteristics required.

In the carding of the conglomerations to create the final product, the fibres can be mixed with in other fibres in order to achieve longevity for the absorbent material when exposed to fluid or alter the material properties.

An absorbent fibre material made according to the method described above provides a product which has the absorbent qualities of cotton wool whilst allowing for on continued exposure to water complete dissolution. This means that in future sewage systems will no longer be blocked by cotton wool and in landfill such products will break breakdown. In simple terms the product could be referred to as cotton wool type material.

The cotton wool type material provides a replacement for cotton wool in all the products made using cotton wool.

Examples made in accordance with the present invention will now be described hereinbelow with reference to the attached drawings, in which:
Figure 1 is a perspective view of a cotton bud according to a first aspect of the present invention;
Figure 2 is a cross-sectional view along Plane A of the cotton bud in Figure 1; and
Figure 3 is a cross-sectional view of the cotton bud in Figure 1 along plane B.

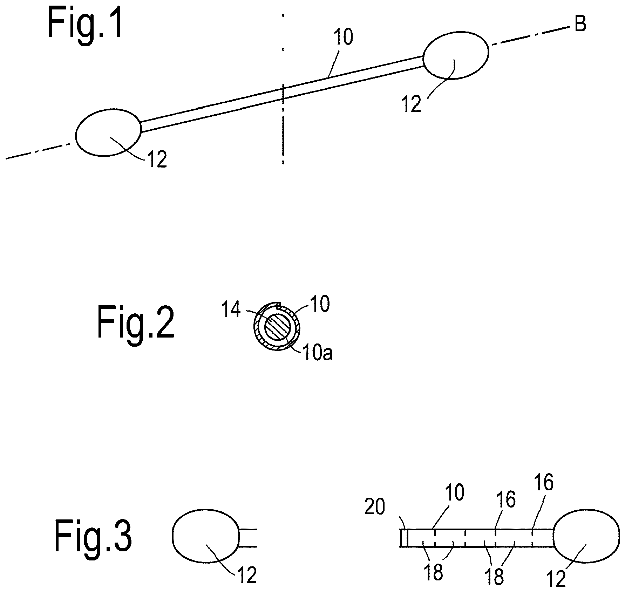

Figure 1 shows a perspective view of a cotton bud comprising a shaft 10 with buds 12 at either end. The buds 12 are secured to the shaft 10 either by external glue or alternatively through attachment to compressed fibre in the shaft 10. The cotton buds 12 are made from entangled dissolvable paper fibres with cotton wool like properties. The shaft 10 is made from rolled dissolvable paper. The fibres of the buds 12 and the paper of the shaft 10 is made preferably from carboxy methyl cellulose and/or wood fibre.

Figure 2 shows a cross-section along plane A in Figure 1 through the shaft 10 of the cotton bud. The shaft 10 comprises rolled dissolvable paper which may have multiple layers with a width of 10a, which are glued together by a water soluble glue. The exterior of the shaft 10 may be coated with a suitable dissolving material to provide extra strength and rigidity. The centre 14 of the shaft 10 is hollow. This can be filled with compressed entangled dissolvable fibres.

Figure 3 shows a cross-sectional view along plane B in Figure 1 of the cotton bud showing the shaft 10 containing a series of compressed buds 18 which are separated by pre-scorings 16 to enable these to be broken when a fresh bud 12 upon use is pulled out of the centre 14 of the shaft 10. There is a break 20 in the middle as both halves of the shaft 10 contain compressed entangled fibre material for the buds 12 at each end.

In use the cotton bud, once the bud 12 becomes soiled can have the bud 12 pulled off pulling out of the shaft 10 a further bud 12. The new bud 12 is separated from the old bud 12 by the pre-scoring 18. Owing to the dissolvable nature of the bud 12, this can easily be disposed of by washing down a sink or a lavatory with no likelihood of blockage.

In manufacture raw material/feedstock namely 10pt (252gsm) Facestock (calendered water soluble paper) comprised of sodium carboxyl methyl cellulose and wood pulp was provided. The feedstock was placed in a 400W food blender with a gearless motor. The packing of the fibre material into the blender was loose to facilitate aeration. The feedstock was blended for 1 minute and allowed to settle. This was repeated twice to produce the required consistency of the product. The product is comprised of loose blended cotton wool type material.

A preferred embodiment of the object is to form 1 inch balls of cotton wool type material, i.e. a cotton wool type ball, which weigh half an ounce.

A cotton wool type ball produced by this method was able to mop up small water spillages without losing form. Once the ball was placed in a glass of water dissolution occurred within 1 minute and with slight agitation complete dissolution was achieved, leaving no large pieces of fibre material.

## Claims

1. A cotton bud, **characterised in that** a shaft (10) is made from rolled dissolvable paper and the buds (12) at the ends comprise entangled dissolvable cotton wool type fibres.

2. A cotton bud according to Claim 1, **characterised in that** the hollow shaft (10) contains further compressed entangled paper fibres which can be pulled out when the existing bud (12) needs to be refreshed when in use.

3. A cotton bud according to Claim 1 or Claim 2, **characterised in that** the compressed fibres have pre-scored breaks (16) in order to break off the used part once the fresh part has been pulled out.

4. A cotton bud according to any preceding Claim, **characterised in that** the dissolvable paper of the shaft (10) is carboxy methyl cellulose.

5. A cotton bud according to any preceding Claim, **characterised in that** the shaft (10) is coated with an appropriate soluble coating in order to provide added stiffness and endurance.

6. A cotton bud according to any one of Claims 1 to 4, **characterised in that** the rolled paper is a multilayer paper held together by dissolvable glue.

## Patentansprüche

1. Wattestäbchen, **dadurch gekennzeichnet, dass** ein Schaft (10) aus aufgerolltem auflösbaren Papier hergestellt ist und die Spitzen (12) an den Enden verwickelte auflösbare watteartige Fasern umfassen.

2. Wattestäbchen nach Anspruch 1, **dadurch gekennzeichnet, dass** der hohle Schaft (10) weitere verdichtete verwickelte Papierfasern enthält, die herausgezogen werden können, wenn die vorhandene Spitze (12) während des Gebrauchs erneuert werden muss.

3. Wattestäbchen nach Anspruch 1 oder Anspruch 2, **dadurch gekennzeichnet, dass** die verdichteten Fasern vorgekerbe Bruchstellen (16) zum Abbrechen des verwendeten Teils nach dem Herausziehen des neuen Teils aufweisen.

4. Wattestäbchen nach einem der vorhergehenden Ansprüche, **dadurch gekennzeichnet, dass** das auflösbare Papier des Schafts (10) Carboxymethylcellulose ist.

5. Wattestäbchen nach einem der vorhergehenden Ansprüche, **dadurch gekennzeichnet, dass** der Schaft (10) mit einer geeigneten löslichen Beschichtung beschichtet ist, um zusätzliche Steifheit und Widerstandsfähigkeit bereitzustellen.

6. Wattestäbchen nach einem der Ansprüche 1 bis 4, **dadurch gekennzeichnet, dass** das aufgerollte Papier ein durch auflösbaren Klebstoff zusammengehaltenes mehrschichtiges Papier ist.

## Revendications

1. Bâtonnet ouaté, **caractérisé en ce qu'**une tige (10) est fabriquée à partir de papier soluble enroulé et les tampons (12) au niveau des extrémités comprennent des fibres enchevêtrées de type ouate soluble.

2. Bâtonnet ouaté selon la revendication 1, **caractérisé en ce que** la tige creuse (10) contient en outre des fibres de papier enchevêtrées comprimées qui peuvent être retirées lorsque le tampon existant (12) doit être rafraîchie lors de l'utilisation.

3. Bâtonnet ouaté selon la revendication 1 ou la revendication 2, **caractérisé en ce que** les fibres comprimées comportent des cassures préétablies (16) afin de casser la partie usagée une fois la partie neuve a été retirée.

4. Bâtonnet ouaté selon une quelconque revendication précédente, **caractérisé en ce que** le papier soluble de la tige (10) est de la carboxyméthylcellulose.

5. Bâtonnet ouaté selon une quelconque revendication précédente, **caractérisé en ce que** la tige (10) est revêtue d'un revêtement soluble approprié afin de fournir une rigidité et une endurance accrues.

6. Bâtonnet ouaté selon l'une quelconque des revendications 1 à 4, **caractérisé en ce que** le papier enroulé est un papier multicouche maintenu ensemble par une colle soluble.
